# EUROPEAN PATENT APPLICATION

(11) **EP 4 552 624 A1**
(43) Date of publication of application: **14.05.2025**
(21) Application number: 23868047.4
(22) Date of filing: 06.09.2023
(51) Int. Cl.: A61F 13/47, A61F 13/475, A61F 13/49, A61F 13/494, A61F 13/53, A61F 13/534, A61F 13/535

(54) **ABSORBENT ARTICLE**

(30) Priority: 20.09.2022 JP 2022149575
(71) Applicant: Unicharm Corporation, Ehime 799-0111 (JP)
(72) Inventor: AKINO, Chieri, Kanonji-shi, Kagawa 769-1602 (JP); NAGAI, Takahito, Kanonji-shi, Kagawa 769-1602 (JP); KAWAMURA, Kouji, Kanonji-shi, Kagawa 769-1602 (JP)
(74) Representative: Dolleymores
(86) International application number: PCT/JP2023/032513
(87) International publication number: WO 2024/062924

(57) **Abstract**

This absorbent article (1) comprises an absorbent core (11a), wherein: the absorbent core (11a) has a central region (11aCV) and side regions (11aSV) on a crotch portion that straddles a central location (CL) of an absorbent body (10) in the longitudinal direction; the absorbent core (11a) has a pair of guides (11G) along the longitudinal direction at boundaries between the central region (11aCV) and the side regions (11aSV) in the left-right direction; the length (L1) of the central region (11aCV) in the left-right direction at the center location (CL) is shorter than the length (L2) of either side region (11aSV) in the left-right direction; and the guides (11G) guide deformation such that sites from the guides (11G) to ends (11ae) of the absorbent core (11a) in the left-right direction face the thighs of the wearer when worn.

## Description

### FIELD

The present invention relates to an absorbent article.

### BACKGROUND

In absorbent articles that include an absorbent body for absorbing excreted fluid such as urine and menstrual blood, a technology is conventionally known in which the absorbent body deforms by bending, for example, when the absorbent article is put on, to improve leak-proofness and fit. For example, Patent Document 1 discloses a disposable diaper including a central absorbent body and a second absorbent body. In such a diaper, when the diaper is put on, a region between the central absorbent body and the second absorbent body is bent, and the second absorbent body is thus bent toward a wearer's thighs side. Patent Document 2 discloses an absorbent article including an absorbent core that has: a central low-basis-weight portion in a central portion of the absorbent core in the width direction; and a pair of side low-basis-weight portions on two sides of the absorbent core in the width direction. In the central low-basis-weight portion, the basis weight of the absorbent core is lower than in the surrounding region. In such an absorbent article, when the absorbent article is put on, a cross-sectional shape of the absorbent core changes into a W shape at two side ends of the central low-basis-weight portion and the side low-basis-weight portions.

### [CITATION LIST]

### [PATENT LITERATURE]

PTL 1: Japanese Patent No. 4229581
PTL 2: Japanese Patent Application Publication No. 2017-217160

### SUMMARY

### [TECHNICAL PROBLEM]

In Patent Document 1, the absorbent article has a boxershorts shape during usage, and has excellent leak-proofness. However, in the case where the user with thick thighs puts on the absorbent article, the narrow crotch width of the user and the long widthwise length of a part of the absorbent article corresponding to the user's crotch cause the absorbent body to be compressed and squashed from widthwise outer sides due to the external pressure of the user's thighs. This reduces the width of the absorbent body to cause a risk of leakage. The absorbent article of Patent Document 2 also prevents the side leakage of body fluid by the absorbent core coming into close contact with the wearer. However, in the case where a plurality of deformation points is provided in the width direction of the absorbent core, widthwise outer portions of the absorbent body may be crumpled at a time of rubbing against the wearer's thick thighs, which makes the absorbent core less likely to maintain the function thereof.

The present invention has been made in view of the circumstances described above, its object is to provide an absorbent article in which an absorbent core is less likely to lose its shape at the crotch and fits to the user's body to suppress leakage, even when a user with thick thighs puts on the absorbent article.

### [SOLUTION TO PROBLEM]

A main aspect of the present invention for achieving the above-described aspect is an absorbent article having a longitudinal direction, a lateral direction, and a thickness direction that intersect each other in an unfolded and stretched state of the absorbent article, the absorbent article including: an absorbent main body including an absorbent core, in a crotch portion that spans a central position of the absorbent main body in the longitudinal direction, the absorbent core having: a central region that is located in a central portion of the absorbent core in the lateral direction; and side regions that are respectively located on two lateral outward sides of the central region, the absorbent core having a pair of guide portions, the pair of guide portions being respectively located at boundaries between the central region and the side regions in the lateral direction, the pair of guide portions extending in the longitudinal direction, at the central position, a lateral length of the central region being shorter than a lateral length of each of the side regions located on the two lateral outward sides of the central region, when the absorbent article is put on, each of the guide portions guiding deformation of a region between each of the guide portions and a lateral end of the absorbent core such that the region faces a thigh of a wearer.

Features of the present invention other than the above will become clear by reading the description of the present specification with reference to the accompanying drawings.

### [ADVANTAGEOUS EFFECT OF THE INVENTION]

According to the present invention, it is possible to provide an absorbent article in which an absorbent core is less likely to lose its shape at a crotch portion and fits to the user's body to suppress leakage, even when a user with thick thighs puts on the absorbent article.

### BRIEF DESCRIPTION OF DRAWINGS

FIG. 1 is a schematic front view of an underpants-shaped disposable diaper 1.
FIG. 2 is a schematic plan view of a diaper 1 in an unfolded and stretched state.
FIG. 3 is a plan view illustrating a configuration of an absorbent main body 10 and leak-proof-wall portions 50.
FIG. 4 is a schematic cross-sectional view taken along a line A-A in FIG. 3.
FIG. 5A is a schematic cross-sectional view illustrating deformation of an absorbent core 11a during usage.
FIG. 5B is an enlarged view of a part of FIG. 3.
FIG. 6 is a schematic plan view of a diaper 1' in an unfolded and stretched state as viewed from a skin side, according to a second embodiment.
FIG. 7 is a plan view illustrating a configuration of an absorbent main body 10 according to the second embodiment.
FIG. 8 is a schematic cross-sectional view taken along a line B-B in FIG. 7.
FIG. 9 is a schematic cross-sectional view illustrating deformation of an absorbent core 110 (111, 112) during usage.

### DESCRIPTION OF EMBODIMENTS

At least the following matters will become clear with the description of this specification and the attached drawings. Aspect 1 is an absorbent article having a longitudinal direction, a lateral direction, and a thickness direction that intersect each other in an unfolded and stretched state of the absorbent article, the absorbent article including: an absorbent main body including an absorbent core, in a crotch portion that spans a central position of the absorbent main body in the longitudinal direction, the absorbent core having: a central region that is located in a central portion of the absorbent core in the lateral direction; and side regions that are respectively located on two lateral outward sides of the central region, the absorbent core having a pair of guide portions, the pair of guide portions being respectively located at boundaries between the central region and the side regions in the lateral direction, the pair of guide portions extending in the longitudinal direction, at the central position, a lateral length of the central region being shorter than a lateral length of each of the side regions located on the two lateral outward sides of the central region, when the absorbent article is put on, each of the guide portions guiding deformation of a region between each of the guide portions and a lateral end of the absorbent core such that the region faces a thigh of a wearer.

According to the absorbent article of Aspect 1, when the absorbent article is put on, the guide portions make the central region of the absorbent core likely to deform into a convex shape toward the skin side, and the width (lateral length) of the central region that deforms into the convex shape toward the skin side is shorter than the width of each side region, thereby making the central region less likely to be squashed by the compressive force from the two lateral sides of the absorbent core. In addition, the regions between the guide portions and lateral ends of the absorbent core such that the regions face wearer's thighs, which can suppress the loss of the shapes of the absorbent core due to rubbing against the thick thighs to prevent leakage.

Aspect 2 is an absorbent article having a longitudinal direction, a lateral direction, and a thickness direction that intersect each other in an unfolded and stretched state of the absorbent article, the absorbent article including: an absorbent main body including an absorbent core, in a crotch portion that spans a central position of the absorbent main body in the longitudinal direction, the absorbent core having: a central region that is located in a central portion of the absorbent core in the lateral direction; and side regions that are respectively located on two lateral outward sides of the central region, the absorbent core having a pair of guide portions, the pair of guide portions being respectively located at boundaries between the central region and the side regions in the lateral direction, the pair of guide portions extending in the longitudinal direction, at the central position, a lateral length of the central region being shorter than a lateral length of each of the side regions located on the two lateral outward sides of the central region, a lateral length of one of the side regions being equal to or less than twice the lateral length of the central region.

According to the absorbent article of Aspect 2, when the absorbent article is put on, the guide portions make the central region of the absorbent core likely to deform into a convex shape toward the skin side, and the width of the central region is shorter than the width of each side region, thereby making the central region less likely to be squashed by the external force from the two lateral sides of the absorbent core. Compared to the case where the width (lateral length) of one of the side regions is more than twice the width of the central region, in the case where the width of one of the side regions is equal to or less than twice the width of the central region, the side regions are less likely to be twisted or deform even when rubbing against the thick thighs occurs during usage.

Aspect 3 is an absorbent article having a longitudinal direction, a lateral direction, and a thickness direction that intersect each other in an unfolded and stretched state of the absorbent article, the absorbent article including: an absorbent main body including an absorbent core, in a crotch portion that spans a central position of the absorbent main body in the longitudinal direction, the absorbent core having: a central region that is located in a central portion of the absorbent core in the lateral direction; and side regions that are respectively located on two lateral outward sides of the central region, the absorbent core having a pair of guide portions, the pair of guide portions being respectively located at boundaries between the central region and the side regions in the lateral direction, the pair of guide portions extending in the longitudinal direction, at the central position, a lateral length of the central region being shorter than a lateral length of each of the side regions located on the two lateral outward sides of the central region, the absorbent core having liquid-absorbent fiber, an existence region referring to a region in the absorbent core where the liquid-absorbent fiber exists, a non-existence region referring to a region in the absorbent core where the liquid-absorbent fiber does not exist, the side regions being constituted by only the existence region.

According to the absorbent article of Aspect 3, when the absorbent article is put on, the guide portions make the central region of the absorbent core likely to deform into a convex shape toward the skin side, and the width of the central region is shorter than the width of each side region, thereby making the central region less likely to be squashed by the external force from the two lateral sides of the absorbent core. The liquid-absorbent fiber existing over the entire side regions increases the stiffness, and thus, the loss of the shape in the absorbent core can be suppressed even when the side regions are rubbed against the thick thighs during usage.

Aspect 4 is the absorbent article according to any one of Aspects 1 to 3, wherein at least the side regions have means to increase stiffness of the absorbent core.

According to the absorbent article of Aspect 4, stiffness of the side regions is increased, which can suppress the loss of the shapes in the side regions due to rubbing against the thick thighs during usage.

Aspect 5 is the absorbent article according to any one of Aspects 1 to 4, wherein the side regions have a compressed portion.

According to the absorbent article of Aspect 5, the compressed portion increases stiffness of the side regions, which can suppress the loss of the shape in the absorbent core due to rubbing against the thick thighs.

Aspect 6 is the absorbent article according to any one of Aspects 1 to 5, wherein a central stretchable member that stretches and contracts in the longitudinal direction is provided in a central portion of the crotch portion in the lateral direction, and the central region has a portion that is overlapped with the central stretchable member as viewed in the thickness direction.

According to the absorbent article of Aspect 6, the central stretchable member makes it easier for the central region to deform toward the skin side (crotch side). Thus, it is easily suppressed that the absorbent core is squashed by the external force from the two lateral sides of the absorbent core during usage.

Aspect 7 is the absorbent article according to Aspect 6, wherein the absorbent core has a constricted portion that is located between two longitudinal end portions of the absorbent core, a lateral length of the constricted portion is shorter than a lateral length of each longitudinal end portion of the absorbent core, and the central stretchable member is provided in a region where the constricted portion exists with respect to the longitudinal direction.

According to the absorbent article of Aspect 7, the constricted portion is a portion corresponding to the wearer's crotch portion when the absorbent article is put on, and the central stretchable member in the region where the constricted portion exists contracts in the longitudinal direction, which can reduce the distance in the constricted portion between the absorbent core and the urination orifice. This can suppress leakage.

Aspect 8 is the absorbent article according to Aspect 6 or 7, wherein a longitudinal one side is a front side of a wearer, a longitudinal other side is a back side of a wearer, and in the central stretchable member, a longitudinal length from a front end of the central stretchable member to the central position is longer than a longitudinal length from a back end of the central stretchable member to the central position.

According to the absorbent article of Aspect 8, the central stretchable member is arranged on the front side, and contraction of the central stretchable member can reduce the distance between the absorbent core and the urination orifice that is located on the wearer's front side (stomach side), thereby making it easier to absorb urine. This can suppress leakage.

Aspect 9 is the absorbent article according to any one of Aspects 1 to 3 and 6 to 8, wherein the absorbent core includes a first absorbent core and a second absorbent core that is provided on a non-skin side in the thickness direction with respect to the first absorbent core, and the side regions include an overlap region where the first absorbent core and the second absorbent core are overlapped with each other, as viewed in the thickness direction.

According to the absorbent article of Aspect 9, in each of the side regions, the stiffness increases in the overlap region where the first absorbent core and the second absorbent core are overlapped with each other, which can suppress the loss of the shape in the absorbent core due to rubbing against the thick thighs.

Aspect 10 is the absorbent article according to Aspect 9, wherein at least one of the first absorbent core and the second absorbent core has a constricted portion between two longitudinal end portions of the one of the first absorbent core and the second absorbent core, a lateral length of the constricted portion is shorter than a lateral length of each of the longitudinal end portions of the one of the first absorbent core and the second absorbent core, the side regions are located in a region where the constricted portion is provided with respect to the longitudinal direction, and stiffness of the overlap region in the side regions is higher than stiffness of a portion other than the constricted portion.

According to the absorbent article of Aspect 10, the overlap region in each of the side regions have high stiffness, which can prevent the loss of the shape in the absorbent core caused by rubbing against the thick thighs.

Aspect 11 is the absorbent article according to Aspect 9 or 10, wherein in the lateral direction of the side regions, a lateral outward end of the first absorbent core is located outside a lateral outward end of the second absorbent core.

According to the absorbent article of Aspect 11, the width (lateral length) of the first absorbent core that is located on the skin side is larger than that of the second absorbent core that is located on the non-skin side. Accordingly, the surface of the first absorbent core close to the wearer's skin can come into close contact with the wearer's thighs, which can make excrement less likely to leak.

Aspect 12 is the absorbent article according to any one of Aspects 9 to 11, wherein the second absorbent core has a constricted portion between two longitudinal end portions of the second absorbent core, a lateral length of the constricted portion is shorter than a lateral length of each of the longitudinal end portions of the second absorbent core, and the first absorbent core is located in a region where the constricted portion is provided with respect to the longitudinal direction.

According to the absorbent article of Aspect 12, the constricted portion is a portion that is likely to be squashed by a lateral external force during usage. Then, the first absorbent core is provided in the region of the constricted portion, which increases the stiffness in the constricted portion. This can make the absorbent core less likely to be squashed.

Aspect 13 is the absorbent article according to any one of Aspects 9 to 12, wherein in the central region, a low-basis-weight portion having a lower basis weight than in a surrounding region is provided in at least one of the first absorbent core and the second absorbent core.

According to the absorbent article of Aspect 13, in the central region that is induced to deform into a convex shape toward the skin side when the absorbent article is put on, the low-basis-weight portion serves as a deformation point, and the low-basis-weight portion makes the central region easily deform into the convex shape toward the skin side.

Aspect 14 is the absorbent article according to any one of Aspects 9 to 13, wherein at the central position, a lateral length of the first absorbent core is longer than a lateral length of the second absorbent core, and, at the central position and on a lateral one side, a lateral length from a lateral outward end of the first absorbent core on the lateral one side to a lateral outward end of the second absorbent core on the lateral one side is shorter than a length L1 that is a lateral length of the central region.

According to the absorbent article of Aspect 14, at the central position, the width of the first absorbent core is larger than that of the second absorbent core, but the difference in width between the first absorbent core and the second absorbent core is shorter than the lateral length L1 of the central region. As a result, in the absorbent core, the second absorbent core also extends in the vicinity of the lateral ends of the absorbent core. Thus, the stiffness is easily maintained even in the lateral ends of the absorbent core. This makes the absorbent core likely to be maintained in a flat shape even during usage, which can suppress leakage.

Aspect 15 is the absorbent article according to any one of Aspects 1 to 14, wherein the absorbent main body has a pair of leak-proof-wall portions on two lateral sides of the absorbent main body, an elastic member that is contractable in the longitudinal direction is arranged in the pair of leak-proof wall portions, and at the central position and on a lateral one side, a lateral length from a lateral outward end to a lateral inward end of the leak-proof-wall portion on the lateral one side is equal to or less than half a lateral length from the lateral outward end of the leak-proof-wall portion on the lateral one side to the guide portion on the lateral one side.

According to the absorbent article of Aspect 15, the leak-proof-wall portion rises up when the absorbent article is put on, but there is a risk that the leak-proof-wall portion covers an absorbent surface of the absorbent main body in the case where the lateral length of the leak-proof-wall portion is excessively long. The lateral length of the leak-proof-wall portion is not excessively long, which can prevent leakage without covering the absorbent surface of the absorbent main body.

Aspect 16 is the absorbent article according to Aspect 15, wherein the absorbent core has a constricted portion between two longitudinal end portions of the absorbent core, a lateral length of the constricted portion is shorter than a lateral length of each of the longitudinal end portions of the absorbent core, the constricted portion has a narrowest portion where the lateral length of the constricted portion is shortest, and at the central position and on a lateral one side, the lateral length from the lateral outward end to the lateral inward end of the leak-proof-wall portion on the lateral one side is shorter than a lateral length from the lateral outward end of the leak-proof-wall portion on the lateral one side to an end of the narrowest portion on the lateral one side.

According to the absorbent article of Aspect 16, at the narrowest portion in the absorbent core, the lateral length of the leak-proof-wall portion is set so as not to reach the narrowest portion in the absorbent core, and thus, the leak-proof-wall portion does not cover the narrowest portion, which can reliably exhibit the absorption performance of the absorbent core. This can prevent leakage.

Aspect 17 is the absorbent article according to any one of Aspects 1 to 16, wherein the absorbent main body includes: a top sheet that is located on a skin side in the thickness direction with respect to the absorbent core; and a second sheet that is provided between the top sheet and the absorbent core, the central region is located in the region where the second sheet is provided with respect to the lateral direction, and the central region has a portion that is overlapped with the second sheet as viewed in the thickness direction.

According to the absorbent article of Aspect 17, the central region is located in the region where the second sheet is provided, which increases the stiffness in the central region. When deformation of the central region is guided during usage of the absorbent article, the central region is less likely to be twisted and is more likely to fit to the wearer's body.

Aspect 18 is the absorbent article according to any one of Aspects 1 to 17, wherein the absorbent article includes a waist portion that is arranged around a wearer's waist, the waist portion includes a waist elastic member that is stretchable and contractable in the lateral direction, the absorbent core includes a first absorbent core and a second absorbent core that is provided on the non-skin side in the thickness direction with respect to the first absorbent core, with respect to the longitudinal direction, the first absorbent core is located at a position spaced apart from the waist elastic member, and with respect to the longitudinal direction, the second absorbent core has a portion that is overlapped with the waist elastic member.

According to the absorbent article of Aspect 18, the absorbent core includes the first absorbent core and the second absorbent core, thereby increasing the stiffness of the absorbent core. However, in the case where the two-layered absorbent core exists in the region where the waist elastic member is provided, there is a risk that the wearer feels discomfort when the absorbent article is tightly fitted to the waist. Since the waist portion is distanced from the wearer's crotch, high absorption performance is not required as compared with that in the crotch. Thus, the single-layered absorbent article (second absorbent core) is configured to have the portion that is overlapped with the waist elastic member, which reduces discomfort while preventing leakage.

### First Embodiment

Hereinafter, an underpants-shaped diaper for adults will be described as an example of an absorbent article according to a first embodiment of the present disclosure. However, the absorbent article according to the present disclosure is also applicable to other types of absorbent article such as an underpants-shaped disposable diaper for infants, a tape-type diaper for infants or adults, a sanitary napkin, and a sanitary short.

### Basic Configuration of Underpants-shaped Disposable Diaper 1

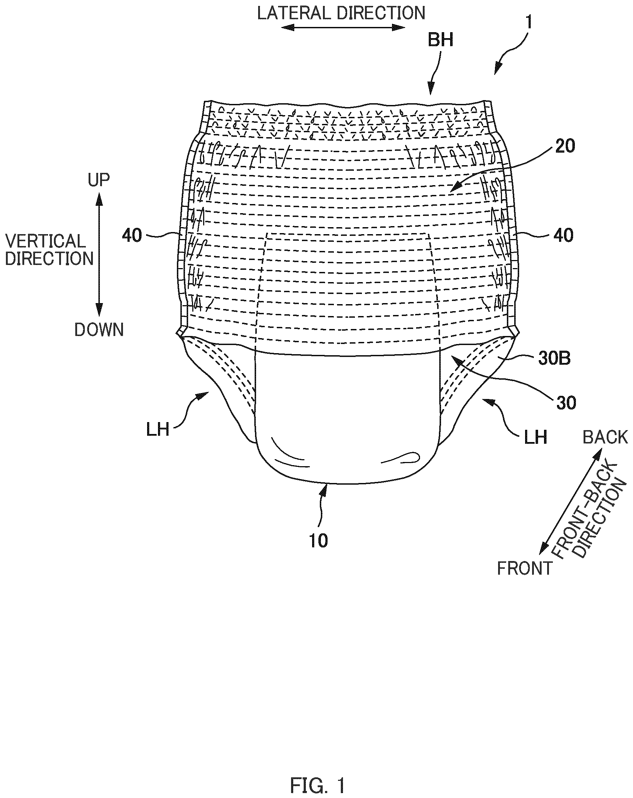

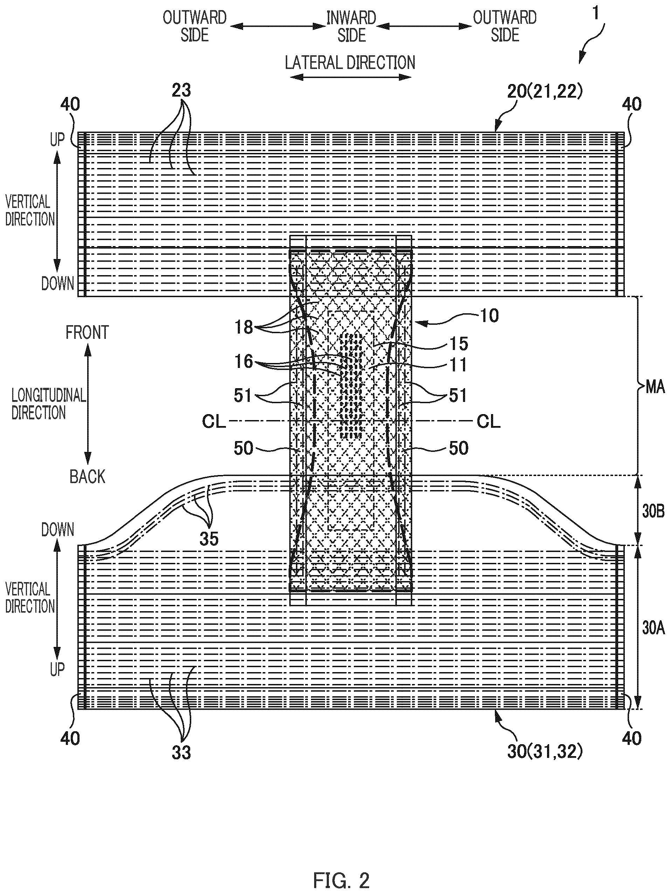

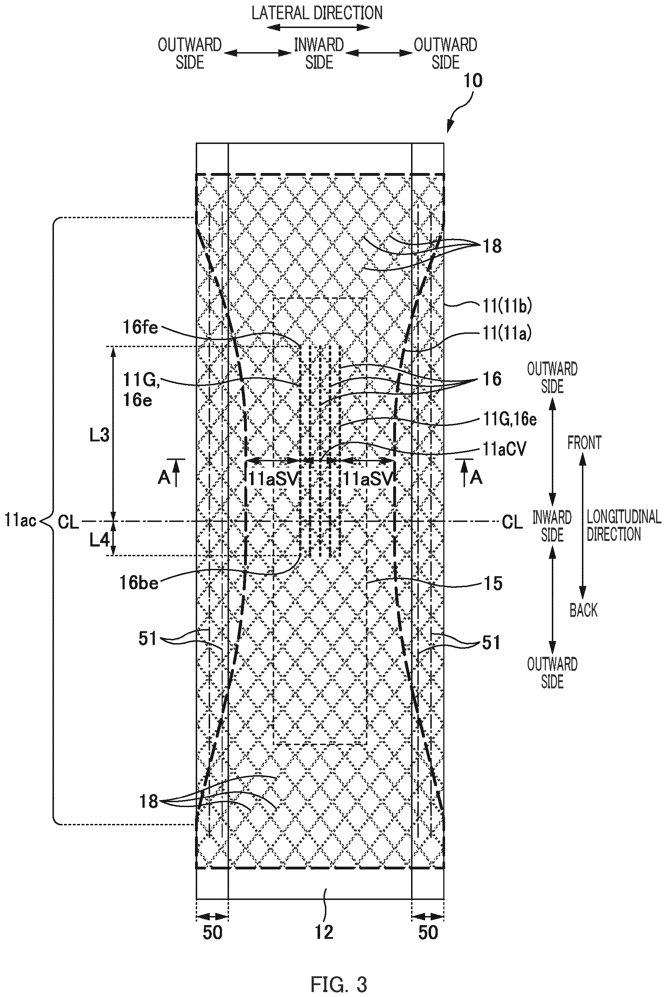

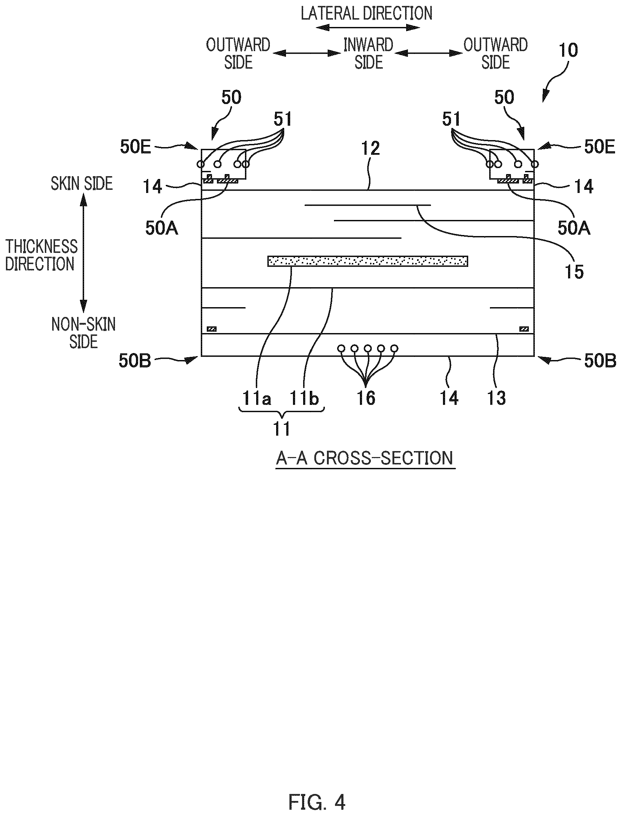

FIG. 1 is a schematic front view of an underpants-shaped disposable diaper 1. FIG. 2 is a schematic plan view of the diaper 1 in an unfolded and stretched state. FIG. 3 is a plan view illustrating a configuration of an absorbent main body 10 and leak-proof-wall portions 50. FIG. 4 is a schematic cross-sectional view taken along a line A-A in FIG. 3. Here, the "unfolded state" in the diaper 1 refers to a state where the diaper 1 is opened in a longitudinal direction by unjoining waist joining portions 40, which will be described later, to separate a pair of waist portions 20 and 30 from each other.

In an underpants-shaped state shown in FIG. 1, the diaper 1 has a vertical direction, a lateral direction, and a front-back direction that intersect each other, and has a waist opening BH and a pair of leg openings LH. In the unfolded state shown in FIG. 2, the diaper 1 has a longitudinal direction, a lateral direction, and a thickness direction that intersect each other. The longitudinal direction is a direction that extends along the vertical direction in the underpants-shaped state. The thickness direction is the direction in which members constituting the diaper 1 are overlaid on each other (see FIG. 4). In the vertical direction, the waist opening BH side is the upper side, and the crotch side is the lower side. In the front-back direction, the stomach side of the wearer is the front side, and the back side of the wearer is the back side. In the thickness direction, the side that comes into contact with the wearer's body (skin) is the skin side, and the side that does not come into contact with the wearer's body (skin) is the non-skin side.

The "stretched state" of the diaper 1 refers to a state in which, by stretching elastic members (e.g., later-described leg elastic members 23, 33 and leak-proof-wall elastic members 51) of the diaper 1, the whole diaper 1 (whole product) has been stretched to eliminate wrinkles, or more specifically, has been stretched such that the dimensions of constituent members of the diaper 1 (e.g., a later-described absorbent main body 10 and waist portions 20, 30) match or are close to the dimensions of such members on their own.

The diaper 1 includes a pair of waist portions 20, 30 that are arranged around a wearer's waist and the absorbent main body 10. Out of the pair of waist portions 20, 30, the one that is placed against the stomach side of the wearer is referred to as the front waist portion 20, and the one that is placed against the back side of the wearer is referred to as the back waist portion 30.

In the diaper 1 in the unfolded state shown in FIG. 2, a longitudinal-one-side end portion of the absorbent main body 10 is placed in the central portion of the front waist portion 20 in the lateral direction, and a non-skin-side surface of the longitudinal-one-side end portion of the absorbent main body 10 and the front waist portion 20 are joined with an adhesive such as a hot-melt adhesive. Similarly, a longitudinal-other-side end portion of the absorbent main body 10 is placed in the central portion of the back waist portion 30 in the lateral direction, and a non-skin-side surface of the longitudinal-other-side end portion of the absorbent main body 10 and the back waist portion 30 are joined with an adhesive such as a hot-melt adhesive. In the diaper 1 in the unfolded state, the absorbent main body 10 is folded one time at a central position CL in the longitudinal direction, and two lateral side portions of the front waist portion 20 and two lateral side portions of the back waist portion 30 are joined in the pair of waist joining portions 40, thereby forming the diaper 1 in an underpants-shaped state. The joining in the waist joining portion 40 is welding or joining with use of an adhesive, for example.

As shown in FIG. 4, the absorbent main body 10 includes an absorbent body 11 that absorbs excreted fluid, a liquid-permeable top sheet 12 that is located on the skin side with respect to the absorbent body 11 in the thickness direction, a liquid-impermeable back sheet 13 that is located on the non-skin side with respect to the absorbent body 11 in the thickness direction, and a sheet member 14. A second sheet 15 is provided between the top sheet 12 and the absorbent body 11. Two lateral side portions of the top sheet 12 are folded back to the non-skin side so as to wrap around the absorbent body 11. A portion of the sheet member 14 is arranged on the non-skin side with respect to the back sheet 13, and other portions of the sheet member 14 form the leak-proof-wall portions 50 on the skin side with respect to the top sheet 12.

The absorbent body 11 includes an absorbent core 11a that absorbs and holds an excreted fluid such as urine, and a liquid-permeable core-wrapping sheet 11b that covers the absorbent core 11a. For example, the absorbent core 11a can be obtained by molding liquid-absorbent fibers such as pulp containing superabsorbent polymer (SAP) into a predetermined shape. On the other hand, the core-wrapping sheet 11b is constituted by tissue paper, for example.

As shown in FIG. 3, the absorbent core 11a of the present embodiment approximately hourglass-shaped in a plan view, and has a constricted portion 11ac that is located between two longitudinal end portions of the absorbent core 11a. The lateral length of the constricted portion 11ac is shorter (the width thereof is narrower) than the lateral length of each longitudinal end portion of the absorbent core 11a. The constricted portion 11ac is a portion sandwiched between the wearer's legs when the diaper 1 is put on, and the lateral length thereof is short (the width thereof is narrow), which makes the absorbent core 11a likely to fit to the wearer's crotch.

As shown in FIGS. 2 and 3, the absorbent body 11, on the entire region thereof, has compressed portions 18 to which embossing is applied. Details of the compressed portions 18 will be described later.

The top sheet 12 is a sheet-like member that covers the absorbent body 11 from the skin side, and is formed of, for example, a liquid-permeable nonwoven fabric sheet whose planar size is larger than that of the absorbent body 11.

The back sheet 13 is a sheet-like member that covers the absorbent body 11 from the non-skin side, and is a liquid-impermeable leak-proof sheet made of polyethylene, polypropylene, or the like. The sheet member 14 is a nonwoven fabric sheet, for example. The second sheet 15 is a sheet-like member that covers at least a part of the absorbent body 11 from the skin side, and is a liquid-permeable nonwoven fabric sheet whose planar size is shorter than that of the absorbent body 11.

The absorbent main body 10 has, on two lateral sides thereof, a pair of leak-proof-wall portions 50 extending in the longitudinal direction of the absorbent main body 10. When the diaper 1 is put on, the leak-proof-wall portions 50 rise from two lateral sides of the absorbent body 11 toward the wearer's skin side. This suppresses leakage of excrement outward from the absorbent main body 10. In the present embodiment, the sheet member 14 that constitutes the exterior of the absorbent main body 10 has the planar size in the lateral direction larger than those of the top sheet 12 and the absorbent core 11a, and thus, in the two lateral ends of the absorbent main body 10, the two lateral side portions of the sheet member 14 are bent toward the skin side in the thickness direction at support points 50B serving as bending points. Then, the two lateral side portions of the sheet member 14 are folded back laterally inward at support points 50E that are located on the skin side with respect to the top sheet 12, and joined to the two lateral end portions of the top sheet 12 in the longitudinal direction at joining portions 50A, to form the leak-proof-wall portions 50.

In each leak-proof-wall portion 50, between the two-layered portions of the sheet member 14, elastic members that are contractible in the longitudinal direction are fixed in the stretched state in the longitudinal direction of the absorbent main body 10. In the present embodiment, a plurality of leak-proof-wall elastic members 51 (e.g., elastic strings) serving as elastic members are arranged in the lateral direction. When the diaper 1 is put on, the leak-proof-wall portions 50 contract in the longitudinal direction of the absorbent main body 10 due to the stretchability of the leak-proof-wall elastic members 51 (elastic members), thereby causing the leak-proof-wall portions 50 to rise toward the wearer's skin side and fit to the wearer's crotch portion.

The leak-proof-wall portions 50 are fixed by front and back fixing portions (not illustrated) provided in front and back end portions of the absorbent main body 10 in the longitudinal direction thereof, in a manner that the leak-proof-wall portions 50 are not capable of rising up. Therefore, the leak-proof-wall portions 50 is capable of rising toward the wearer's skin side, in a region between the front fixing portions and the back fixing portions in the longitudinal direction of the absorbent main body 10. In the leak-proof-wall elastic members 51 of the leak-proof-wall portions 50 shown in FIGS. 2 and 3, only portions that exhibit the stretchability are shown.

In the present embodiment, as shown in FIGS. 2 and 3, in the absorbent body 11, the absorbent core 11a has a plurality of linear compressed portions 18. Specifically, in these "compressed portions" of the present embodiment, the absorbent body 11 (the absorbent core 11a and core-wrapping sheet 11b) has been compressed in the thickness direction by embossing. There are also a plurality of substantially diamond-shaped inner regions that are surrounded on all sides by the compressed portions 18. Due to such compression, the fiber density of the absorbent core 11a is higher than that of uncompressed portions. Note that the compressed portions 18 of the present embodiment are shown by dashed lines in FIGS. 2 and 3, but this is for convenience in the description, and because the absorbent body 11 is actually embossed as described above, the compressed portions 18 are not clearly visible from the skin-side surface of the diaper 1.

Although the compressed portions 18 of the present embodiment are formed linearly, these linear compressed portions 18 may be each formed by a set of dot-like (circular) compressed portions. Alternatively, dot-like compressed portions or linear compressed portions may be formed discretely in a predetermined region. Also, as one example of a formation patterns for the compressed portions 18, the compressed portions 18 may be arranged so as to look like a lattice pattern (so-called quilting pattern) overall as shown in FIG. 3, but other patterns may be used. Also, the lines that form the lattice pattern may be straight lines as shown in FIG. 3, or may be curved lines.

In the compressed portions 18 of the present embodiment, the absorbent core 11a and the core-wrapping sheet 11b are each in a state of being compressed in the thickness direction, but at least the absorbent core 11a is only required to be compressed in the thickness direction. Alternatively, the absorbent core 11a, the core-wrapping sheet 11b, and the top sheet 12 may be compressed in the thickness direction, for example.

The absorbent main body 10 has, in the central portion thereof in the lateral direction and lengthwise direction, a plurality of central stretchable members 16 (e.g., elastic strings) that stretches and contracts in the longitudinal direction. Specifically, the central stretchable members 16 in a state of being stretched in the longitudinal direction are arranged at intervals in the lateral direction and attached to a position between the back sheet 13 and the sheet member 14 in the thickness direction. Only portions of the central stretchable members 16 that exhibit the stretchability are shown in the drawings. The longitudinal length of each central stretchable member 16 is only required to be provided at least in the crotch portion (a crotch portion MA which will be described later) that faces the wearer's crotch. The central stretchable members 16 are not limited to being formed of a string-shaped elastic member such as an elastic string. Alternatively, for example, a plurality of bandshaped elastic strings, stretchy sheets (stretchable films, stretchable nonwoven fabric sheets), or the like may be arranged at intervals in the lateral direction, or a single stretchy sheet with a predetermined dimension may be arranged.

The front waist portion 20 and the back waist portion 30 have: inner-layer sheets 21, 31; outer-layer sheets 22, 32 that are arranged on the non-skin side with respect to the inner-layer sheets 21, 31; and a plurality of waist elastic members 23, 33 that is stretchable and contractable in the lateral direction, respectively. As shown in FIG. 2, in the diaper 1, the region between the front waist portion 20 and the back waist portion 30 is referred to as the crotch portion MA. The crotch portion MA is a part corresponding to the wearer's crotch when the diaper 1 is put on.

The inner-layer sheets 21, 31 and the outer-layer sheets 22, 32 are located on the non-skin side with respect to the absorbent main body 10. The inner-layer sheets 21, 31 and the outer-layer sheets 22, 32 can be constituted by an SMS nonwoven fabric sheet (spunbond/meltblown/spunbond nonwoven fabric sheet), a spunbond nonwoven fabric sheet, an air-through nonwoven fabric sheet, a plastic sheet, a porous plastic sheet, or a laminate sheet including layers of any of the same.

The waist elastic members 23, 33 are arranged in the vertical direction in an underpants-shaped state, at a position between the inner-layer sheets 21, 31 and the outer-layer sheets 22, 32, and are fixed in a state of being stretched in the lateral direction. Thus, the front waist portion 20 and the back waist portion 30 fit to the wearer's waist. The waist elastic members 23, 33 can be constituted by a string-like elastic member made of rubber, spandex, or the like. A cover sheet (not shown) that covers the upper end portion of the absorbent main body 10 from the skin side may be provided.

In the unfolded and stretched state in FIG. 2, the front waist portion 20 is rectangular in a plan view. The back waist portion 30 has an overlap portion 30A that is overlapped with the front waist portion 20 in the front-back direction of the diaper 1 in an underpants-shaped state, and an extension portion 30B that extends downward with respect to the front waist portion 20. The overlap portion 30A is rectangular in a plan view, and the extension portion 30B is shaped as an inverted trapezoid in a plan view. The extension portion 30B can cover the wearer's buttocks. On the other hand, the front waist portion 20 has a shorter vertical length than the back waist portion 30, and does not extend to the vicinity of the wearer's crotch side. For this reason, leg movement is not hindered when the wearer lifts their legs toward their stomach while walking for example, and the wearer walks easily.

Also, extension-portion elastic members 35 are provided along the lower end of the extension portion 30B of the back waist portion 30. As with the waist elastic member 33, the extension-portion elastic members 35 are fixed in a stretched state between the inner-layer sheet 31 and the outer-layer sheet 32. Due to the extension-portion elastic members 35, the extension portion 30B fits to the wearer's buttocks, and curling of the extension portion 30B is thus suppressed. However, the configuration is not limited thereto. For example, similarly to the front waist portion 20, the back waist portion 30 may be rectangular in a plan view, and need not include the extension-portion elastic members 35.

In the case where a disposable diaper such as the diaper 1 of the present embodiment is used, when the diaper is put on or during usage of the diaper, the portion corresponding to the wearer's crotch (in the present embodiment, the crotch portion MA) may be subjected to external forces from the wearer's thighs, and the like, thereby causing the absorbent body (absorbent core) to lose its shape. In particular, in the case where the user has thick thighs, the narrow crotch width causes a risk that the two lateral sides of the absorbent core are strongly pressed to compress and squash the width of the absorbent core, as well as a risk that leakage is caused by the damage to the absorbent core due to rubbing against the thick thighs during usage of the diaper.

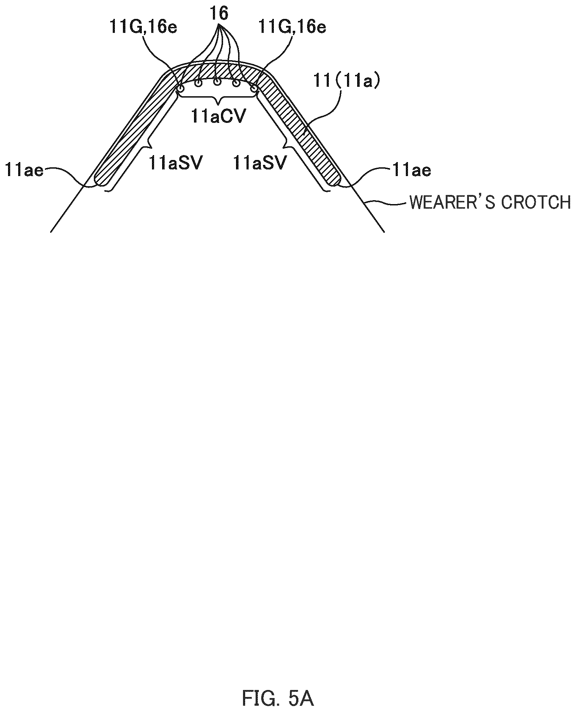

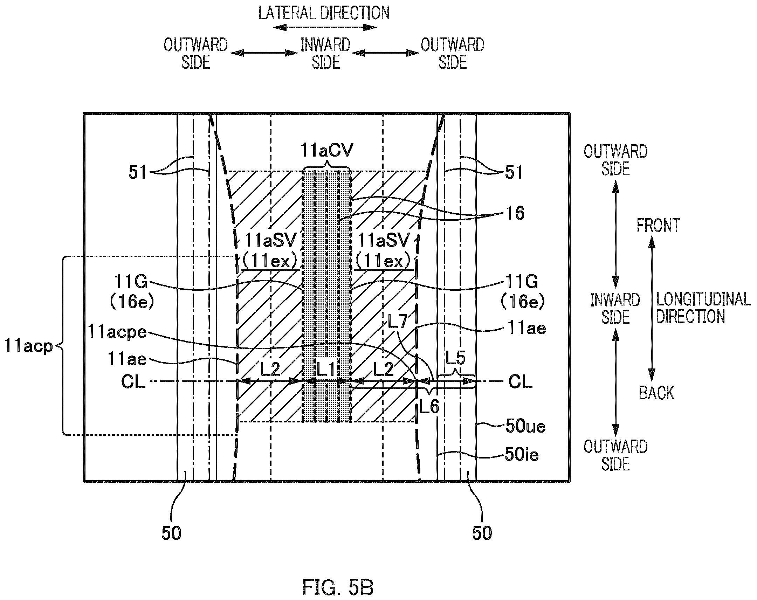

In this regard, the diaper 1 of the present embodiment has the following configuration of the crotch portion MA (FIG. 2) that corresponds to the wearer's crotch and spans the central position CL (FIG. 3) of the absorbent main body 10 in the longitudinal direction thereof. FIG. 5A is a schematic cross-sectional view illustrating deformation of the absorbent core 11a during usage, and FIG. 5B is an enlarged view of a part of FIG. 3. As shown in FIGS. 3 and 5B, the absorbent core 11a of the present embodiment has: a central region 11aCV (a fine dotted region in FIG. 5B) that is located in the central portion of the absorbent core 11a in the lateral direction; and side regions 11aSV (hatched regions in FIG. 5B) that are respectively located on two lateral outward sides of the central region 11aCV. The diaper 1 includes the plurality of central stretchable members 16 in the central portion of the crotch portion MA in the lateral direction, and the central region 11aCV has a portion that is overlapped with the central stretchable members 16 as viewed in the thickness direction. Specifically, in the present embodiment, out of the plurality of central stretchable members 16, two central stretchable members 16e, 16e are located at two lateral ends of the central stretchable members 16, and their respective positions correspond to the positions of the two lateral ends of the central region 11aCV. In other words, the positions of the central stretchable members 16e, 16e are the boundaries between the central region 11aCV and the side regions 11aSV in the lateral direction.

The central stretchable members 16 are each bonded together with the absorbent body 11 via the back sheet 13 with use of an adhesive or the like, and the portion of the absorbent core 11a that is overlapped with the central stretchable members 16 in a plan view in the thickness direction contracts in the longitudinal direction and thus easily deforms so as to be in close contact with the wearer. That is, the central stretchable members 16 make it easier for the central region 11aCV to deform toward the skin side (crotch side) (see FIG. 5A). In this way, the absorbent core 11a has a pair of guide portions 11G, 11G that extend in the longitudinal direction at the boundaries between the central region 11aCV and the side regions 11aSV in the lateral direction, and the guide portions 11G, 11G guide deformation of the absorbent core 11a toward the skin side in the thickness direction. This can suppress that the portion of the central region 11aCV of the absorbent core 11a is squashed due to compressive force in the lateral direction when the diaper 1 is put on. In the present embodiment, the pair of guide portions 11G, 11G correspond to the two central stretchable members 16e, 16e that are located on the two lateral ends of the plurality of central stretchable members 16. In the case where the central stretchable members 16 are each a single stretchy sheet having a predetermined dimension, for example, two lateral ends of such a stretchy sheet serve as the pair of guide portions 11G, 11G. In the case where a plurality of bandshaped elastic strings or a plurality of stretchy sheets are arranged, two lateral outward ends of the elastic strings or sheets located at two lateral ends thereof serve as the pair of guide portions 11G, 11G.

In the present embodiment, out of the central stretchable members 16, the two central stretchable members 16e, 16e that are located at the two lateral ends of the central stretchable members 16 correspond to the pair of guide portions 11G, 11G, but the guide portions 11G are not limited to being constituted by the two lateral outward ends of the central stretchable members. The guide portions 11G can be constituted in any form in which the absorbent core 11a has the difference in stiffness. For example, the central region 11aCV may be constituted by a low-basis-weight portion that has a lower basis weight than in the surrounding region, and two lateral ends of the low-basis-weight portion may be defined as the guide portions 11G, 11G. Alternatively, in the present embodiment, the compressed portions 18 are provided over the entire absorbent body 11, but, for example, compressed portions may be provided such that the difference in stiffness occurs between the central region 11aCV and the side regions 11aSV of the absorbent core 11a to define the regions where the difference in stiffness occurs (i.e., the boundaries between the central region 11aCV and the side regions 11aSV) as the guide portions 11G.

In the present embodiment, as shown in FIG. 5B, at the central position CL of the absorbent main body 10 in the longitudinal direction thereof, a lateral length L1 of the central region 11aCV is shorter than a lateral length L2 of each side region 11aSV located on the two outward sides of the central region 11aCV. Due to reduction of the width of the central region 11aCV (lateral length), the central region 11aCV is less likely to be squashed by the external force from the two lateral sides when the diaper 1 is put on or during usage. As shown in FIGS. 5A and 5B, when the diaper 1 is put on, the pair of guide portions 11G, 11G guide deformation of the regions between the guide portions 11G and lateral ends 11ae of the absorbent core 11a (i.e., the side regions 11aSV) such that the regions face the wearer's thighs. Due to promotion of such deformation, the side regions 11aSV fit along the wearer's thighs, which can suppress loss of the shape in the absorbent core 11a caused by rubbing against the wearer's thighs to prevent leakage.

In addition, it is desirable that the lateral length L2 of one of the side regions 11aSV is equal to or less than twice the lateral length L1 of the central region 11aCV. Compared to the case where the width (lateral length) of one of the side regions 11aSV is more than twice the width of the central region 11aCV, in the case where the width of one of the side regions 11aSV is equal to or less than twice the width of the central region 11aCV, the side regions 11aSV are less likely to be twisted or deform even when rubbing against the thick thighs occurs during usage. Also, it is preferable that the lateral length L2 of one of the side regions 11aSV is equal to or less than 1.5 times the lateral length L1 of the central region 11aCV. In the case where the lateral length of one of the side regions 11aSV is excessively long, there is a risk that such a side region 11aSV serves as a further deformation point of the absorbent core 11a, but the reasonable length is easier to maintain a fitted state to the thighs.

The absorbent core 11a has the liquid-absorbent fibers. In the present embodiment, an existence region 11ex refers to a region in the absorbent core 11a where the liquid-absorbent fibers exist, and a non-existence region refers to a region in the absorbent core 11a where the liquid-absorbent fibers do not exist. Then, the side regions 11aSV are constituted by only the existence region 11ex (FIG. 5B). The liquid-absorbent fibers existing over the entire side regions 11aSV increase the stiffness, and thus, the loss of the shape in the absorbent core 11a can be suppressed even when the side regions 11aSV are rubbed against the thick thighs during usage of the diaper 1.

As described above, the side regions 11aSV do not include the non-existence region where the liquid-absorbent fibers do not exist, which increases the stiffness. However, the configuration is not limited thereto. That is, at least the side regions 11aSV are only required to have the means to increase the stiffness of the absorbent core 11a. In the present embodiment, the side regions 11aSV further have the compressed portions 18. The compressed portions 18 increase the stiffness in the side regions 11aSV, which can further suppress the loss of the shapes in the side regions 11aSV due to rubbing against the thick thighs during usage of the diaper 1.

The absorbent core 11a has the constricted portion 11ac, but the central stretchable members 16 are provided in the region where the constricted portion 11ac exists with respect to the longitudinal direction. The constricted portion 11ac of the absorbent core 11a is a portion corresponding to the crotch portion when the diaper 1 is put on, and each central stretchable member 16 in the region where the constricted portion 11ac exists contracts in the longitudinal direction, which can reduce the distance in the constricted portion 11ac between the absorbent core 11a and the urination orifice. This can suppress leakage.

As shown in FIG. 3, in the central stretchable members 16, a longitudinal length L3 from a front end 16fe to the central position CL is longer than a longitudinal length L4 from a back end 16be to the central position CL. That is, the central stretchable members 16 are arranged on the front side in the longitudinal direction. Positioning of the central stretchable members 16 on the front side in the above-described manner and contraction of the central stretchable members 16 can reduce the distance between the absorbent core 11a and the urination orifice that is located on the wearer's front side (stomach side), thereby making it easier to absorb urine. This can suppress leakage.

As shown in FIG. 5B, at the central position CL and on a lateral one side of the absorbent main body 10, a lateral length L5 is equal to or less than half a lateral length L6. The lateral length L5 refers to a length from a lateral outward end 50ue to a lateral inward end 50ie of the leak-proof-wall portion 50 on the lateral one side, and the lateral length L6 refers to a length from the lateral outward end 50ue of the leak-proof-wall portion 50 on the lateral one side to the guide portion 11G on the lateral one side. The leak-proof-wall portions 50 rise up when the diaper 1 is put on, but there is a risk that the leak-proof-wall portions 50 cover an absorbent surface of the absorbent main body 10 in the case where the lateral length of the leak-proof-wall portions 50 is excessively long. The lateral length of the leak-proof-wall portion 50 is equal to or less than half the lateral length L6 from the outward end 50ue to the guide portion 11G as described above, which can prevent leakage without covering the absorbent surface of the absorbent main body 10.

As shown in FIG. 5B, the constricted portion 11ac in the absorbent core 11a has a narrowest portion 11acp where the lateral length of the constricted portion 11ac is shortest. At the central position CL and on the lateral one side, the lateral length L5 from the lateral outward end 50ue to the lateral inward end 50ie of the leak-proof-wall portion 50 on the lateral one side is shorter than a lateral length L7 from the lateral outward end 50ue of the leak-proof-wall portion 50 on the lateral one side to an end 11acpe of the narrowest portion 11acp on the lateral one side (L5 < L7). At the narrowest portion 11acp of the constricted portion 11ac in the absorbent core 11a, the width of the leak-proof-wall portion 50 (lateral length L5) is set so as not to reach the narrowest portion 11acp of the absorbent core 11a, and thus, the leak-proof-wall portion 50 does not cover the narrowest portion 11acp of the absorbent core 11a, which can reliably exhibit the absorption performance of the absorbent core 11a. This can prevent leakage.

In the present embodiment, the absorbent main body 10 includes the second sheet 15 located between the top sheet 12 and the absorbent core 11a. However, the central region 11aCV is located in the region where the second sheet 15 is provided with respect to the lateral direction, and has the portion that is overlapped with the second sheet 15 as viewed in the thickness direction. Specifically, in the present embodiment, as viewed in the thickness direction, the entire central region 11aCV is the portion that is overlapped with the second sheet 15. The central region 11aCV is located in the region where the second sheet 15 is provided, which increases the stiffness in the central region 11aCV. When deformation of the central region 11aCV toward the skin side is guided during usage of the diaper 1 as shown in FIG. 5A, the central region 11aCV is less likely to be twisted and is more likely to fit to the wearer's body.

The central region 11aCV has, as viewed in the thickness direction, the portion overlapped with the central stretchable members 16 that stretch and contract in the longitudinal direction, but it is preferable that the central region 11aCV does not include the stretchable members that stretch and contract in the lateral direction. When the absorbent core 11a stretches and contracts in the lateral direction, the absorbent core 11a is likely to be squashed. Thus, it is desirable that the central region 11aCV does not have stretchability in the lateral direction.

### Second Embodiment

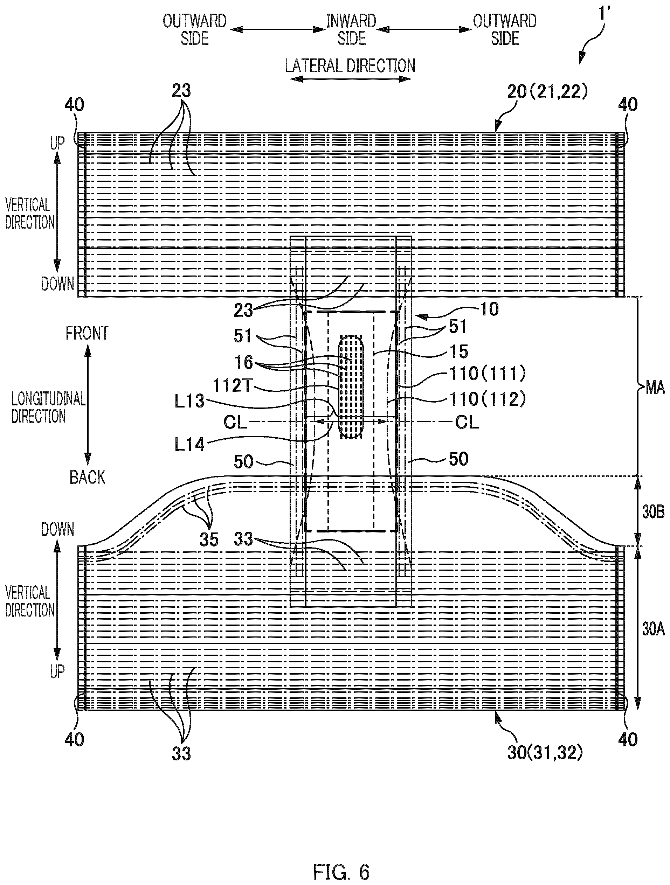

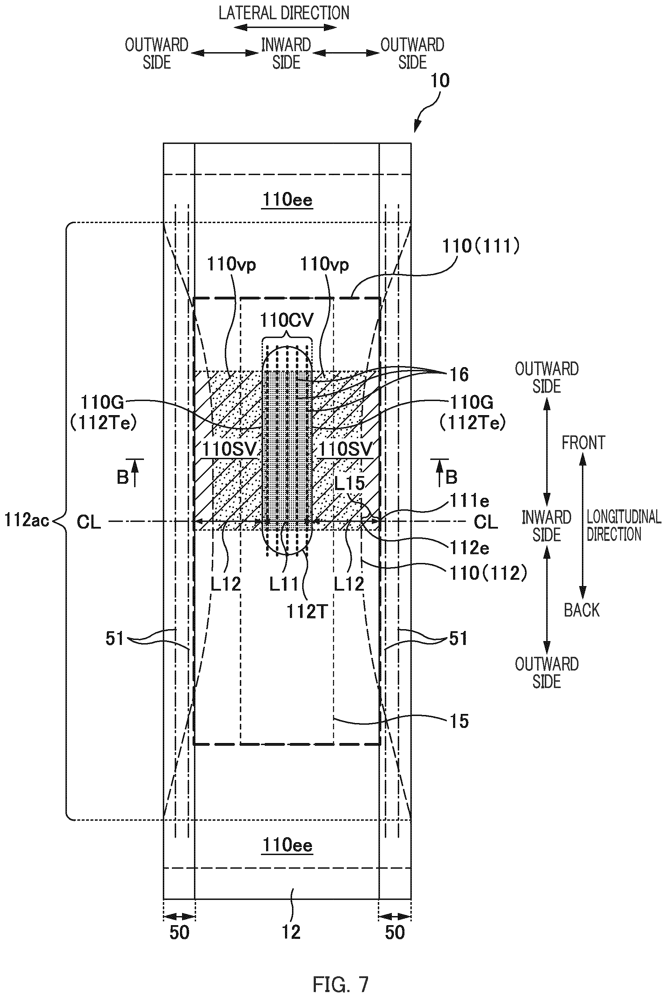

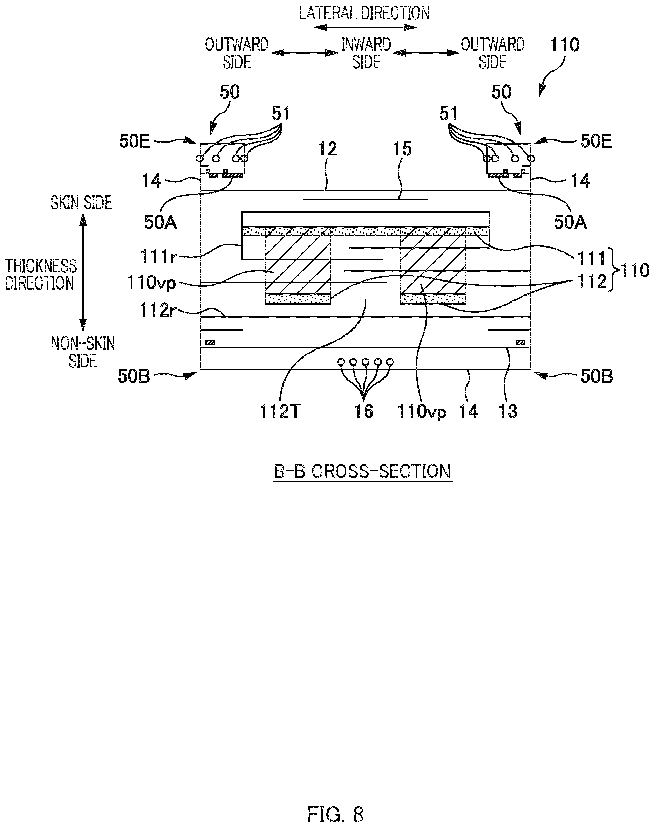

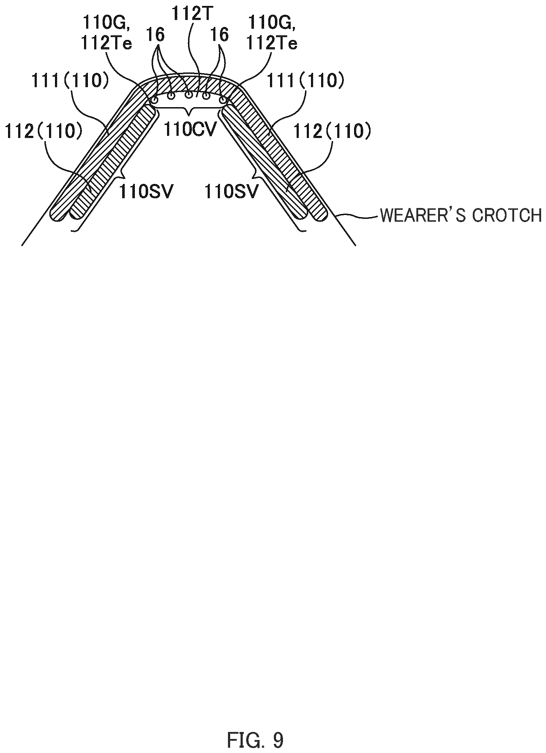

FIG. 6 is a schematic plan view of a diaper 1' in an unfolded and stretched state as viewed from the skin side, according to a second embodiment of the present disclosure. FIG. 7 is a plan view illustrating a configuration of an absorbent main body 10 according to the second embodiment. FIG. 8 is a schematic cross-sectional view taken along a line B-B in FIG. 7. FIG. 9 is a schematic cross-sectional view illustrating deformation of an absorbent core 110 (111, 112) during usage. The "unfolded state" and "stretched state" of the diaper 1' are the same as those described in the first embodiment.

In the first embodiment, the absorbent main body 10 of the diaper 1 includes the single-layered absorbent core 11a, but in the second embodiment, the absorbent main body 10 of the diaper 1' includes a two-layered absorbent core 110. The absorbent core 110 includes a first absorbent core 111 and a second absorbent core 112 that is provided on the non-skin side in the thickness direction with respect to the first absorbent core 111. In the second embodiment, the first absorbent core 111 and the second absorbent core 112 each have a configuration in which the compressed portions 18 are not provided, but the second absorbent core 112 has a low-basis-weight portion (low-basis-weight portion 112T which will be described later) that has a lower basis weight than in the surrounding region. The difference between the first and second embodiments is mainly in the points described above, and other points are substantially the same. Accordingly, the following description will mainly explain these different points, and the same reference numerals are given to the same components and the description thereof is omitted.

### Absorbent Core 110

As shown in FIGS. 6 to 8, in the diaper 1' of the second embodiment, the absorbent main body 10 includes the two-layered absorbent core 110. Specifically, the absorbent core 110 includes the first absorbent core 111 that absorbs and holds an excreted fluid such as urine, and the second absorbent core 112 that is provided on the non-skin side in the thickness direction with respect to the first absorbent core 111 and also absorbs and holds the excreted fluid such as urine. The first absorbent core 111 is covered with a liquid-permeable core-wrapping sheet 111r. Specifically, the core-wrapping sheet 111r is wrapped in the lateral direction, thereby covering the entire outer surface of the first absorbent core 111, but the configuration is not limited thereto.

The second absorbent core 112 is similarly covered with a liquid-permeable core-wrapping sheet 112r. Specifically, as with the first absorbent core 111, the core-wrapping sheet 112r is wrapped in the lateral direction, thereby covering the entire outer surface of the second absorbent core 112, but the configuration is not limited thereto. Liquid-absorbent materials constituting the first absorbent core 111 and the second absorbent core 112, and materials constituting the core-wrapping sheets 111r and 112r are similar to those constituting the absorbent core 11a and the core-wrapping sheet 11b of the first embodiment.

As shown in FIG. 7, the second absorbent core 112 is formed by stacking the liquid-absorbent materials in the thickness direction such that the second absorbent core 112 is approximately hourglass-shaped in a plan view. That is, the second absorbent core 112 has a constricted portion 112ac between two longitudinal end portions of the second absorbent core 112. The lateral width of the constricted portion 112ac is shorter than the lateral width of each longitudinal end portion of the second absorbent core 112.

In the present embodiment, the second absorbent core 112 has, in the central portion thereof in the lateral direction, the low-basis-weight portion 112T that extends in the longitudinal direction and is a portion in which the second absorbent core 112 is hollowed out. In the present embodiment, the second absorbent core 112 is absent in the low-basis-weight portion 112T (i.e., the basis weight is set to zero), but the configuration is not limited thereto. Here, the basis weight is the mass per unit area, and in the present embodiment, the low-basis-weight portion 112T is only required to be a portion where the basis weight is lower than in the surrounding region. Since the absorbent core 110 has the low-basis-weight portion 112T, the absorbent core 110 can be induced to deform at the low-basis-weight portion 112T so as to fit to the wearer's crotch portion. When the wearer urinates, urine can be quickly dispersed in the longitudinal direction along the low-basis-weight portion 112T. Note that the number, arrangement, and shape of the low-basis-weight portion 112T are not limited to the configuration of the low-basis-weight portion 112 of the present embodiment.

As shown in FIG. 7, in the second embodiment, a central region 110CV located in the central portion of the absorbent core 110 in the lateral direction corresponds to a range where the above-mentioned low-basis-weight portion 112T exists in the lateral direction (a fine dotted region in FIG. 7), and two lateral outward sides of the central region 110CV correspond to side regions 110SV (hatched regions in FIG. 7). In the second embodiment, the absorbent main body 10 also includes the plurality of central stretchable members 16 in the central portion thereof in the lateral direction and the lengthwise direction, but two lateral ends 112Te, 112Te of the low-basis-weight portion 112T are located outward in the lateral direction relative to the two lateral ends of the plurality of central stretchable members 16. Thus, in the present embodiment, in the low-basis-weight portion 112T, the two lateral ends 112Te, 112Te extending in the longitudinal direction serve as a pair of guide portions 110G, 110G. In the second embodiment, the absorbent core 110 has a two-layered structure, but the central region 110CV and the side regions 110SV of the present embodiment refer to the region including both the first absorbent core 111 and the second absorbent core 112.

As described above, the absorbent core 110 of the present embodiment has the pair of guide portions 110G, 110G that extend in the longitudinal direction at the boundaries between the central region 110CV and the side regions 110SV in the lateral direction. Due to the contraction of the central stretchable members 16 in the longitudinal direction, the central region 110CV deforms at the guide portions 110G, 110G toward the skin side in the thickness direction and fits to the wearer's crotch, as shown in FIG. 9. Also in the present embodiment, as shown in FIG. 7, at the central position CL of the absorbent main body 10 in the longitudinal direction thereof, a lateral length L11 of the central region 110CV is shorter than a lateral length L12 of each side region 110SV. Accordingly, even when the wearer with thick thighs puts on the diaper 1', the central region 110CV is less likely to be squashed by the compressive force from the two lateral sides. As shown in FIG. 9, when the diaper 1' is put on, the guide portions 110G guide the deformation of the side regions 110SV such that the side regions 110SV face the wearer's thighs, thereby making the side regions 110SV fit to the thighs. Accordingly, in the side regions 110SV, it is suppressed that the shape in the absorbent core 110 is lost in a crumpling manner in the lateral direction due to rubbing against the wearer's thick thighs.

As shown in FIG. 8, the side regions 110SV include overlap regions 110vp, respectively, where the first absorbent core 111 and the second absorbent core 112 are overlapped with each other, as viewed in the thickness direction. In the side regions 110SV, the stiffness increases in the overlap regions 110vp (coarse dotted region in FIG. 7) where the first absorbent core 111 and the second absorbent core 112 are overlapped with each other, which can suppress the loss of the shape in the absorbent core 110 due to rubbing against the thick thighs.

As described above, in the present embodiment, the second absorbent core 112 has the constricted portion 112ac, and the side regions 110SV are located in the region where the constricted portion 112ac is provided with respect to the longitudinal direction. The above-mentioned overlap regions 110vp (FIG. 7) where the first absorbent core 111 and the second absorbent core 112 are overlapped with each other in the side regions 110SV are high-stiffness portions where stiffness is higher than that in a portion 110ee (FIG. 7) other than the constricted portion 112ac.

The stiffness values of the overlap regions 110vp and the portion 110ee other than the constricted portion 112ac can be obtained by, for example, measuring Gurley Stiffness values and dividing such values by the length of a sample piece. The Gurley stiffness can be measured in accordance with JIS-L1096, for example, using a No. 311 Gurley stiffness tester manufactured by Yasuda Seiki Seisakusho, Ltd. Note that in the present embodiment, it is sufficient to be able to check the magnitude relationship of the stiffness between the overlap regions 110vp and the portion 110ee, and therefore, it is not necessary to obtain an exact value, and a certain level of error in the measured values is permissible.

The constricted portion 112ac is a portion corresponding to the wearer's crotch, and the side regions 110SV that are provided in the region of the constricted portion 112ac are regions where rubbing against the wearer's thick thighs often occurs when the diaper 1' is put on. The overlap regions 110vp in the side regions 110SV have high stiffness, which can prevent the loss of the shape in the absorbent core 110 caused by rubbing against the thick thighs. In the present embodiment, the second absorbent core 112 has the constricted portion 112ac, but at least one of the first absorbent core 111 and the second absorbent core 112 is only required to have the constricted portion.

In the present embodiment, the first absorbent core 111 is also located in the region where the constricted portion 112ac is provided with respect to the longitudinal direction. The constricted portion 112ac is a portion that is likely to be squashed by a lateral external force during usage of the diaper 1'. Then, the first absorbent core 111 is provided in the region of the constricted portion 112ac, which increases the stiffness in the constricted portion 112ac. This can make the absorbent core 110 less likely to be squashed.

As shown in FIG. 7, in the lateral direction of the side regions 110SV, a lateral outward end 111e of the first absorbent core 111 is located outside a lateral outward end 112e of the second absorbent core 112. That is, the width (lateral length) of the first absorbent core 111 that is located on the skin side is larger than that of the second absorbent core 112 that is located on the non-skin side. With such a configuration, the surface of the first absorbent core 111 close to the wearer's skin can come into close contact with the wearer's thighs, which can make excrement less likely to leak.

As described above, in the present embodiment, the central region 110CV has the low-basis-weight portion 112T in the second absorbent core 112. In the central region 110CV that is induced to deform into a convex shape toward the skin side when the diaper 1' is put on, the low-basis-weight portion 112T serves as a deformation point, and the low-basis-weight portion 112T makes the central region 110CV easily deform into the convex shape toward the skin side. In the present embodiment, the low-basis-weight portion 112T is provided in the second absorbent core 112, but the configuration is not limited thereto. The low-basis-weight portion 112T is simply required to be provided in at least one of the first absorbent core 111 and the second absorbent core 112T.

As shown in FIG. 6, at the central position CL, a lateral length L13 of the first absorbent core 111 is longer than a lateral length L14 of the second absorbent core 112. That is, at the central position CL, the width (lateral length) of the first absorbent core 111 is larger than that of the second absorbent core 112. As shown in FIG. 7, when the lateral length of the central region 110CV refers to the lateral length L11, at the central position CL and on the lateral one side, a length L15 from the lateral outward end 111e of the first absorbent core 111 on the lateral one side to the lateral outward end 112e of the second absorbent core 112 on the lateral one side is shorter than the above-mentioned lateral length L11. The above-mentioned length L15 is the difference in width between the first absorbent core 111 and the second absorbent core 112 at the central position CL, and the difference in width between the first absorbent core 111 and the second absorbent core 112 (L15) is shorter than the lateral length L11. As a result, in the absorbent core 110, the second absorbent core 112 also extends in the vicinity of the lateral ends of the absorbent core 110. Thus, the stiffness is maintained even in the lateral ends of the absorbent core 110, which makes the absorbent core 110 likely to be maintained in a flat shape even during usage. This can suppress side leakage and the like.

Returning to FIG. 6, in the present embodiment, with respect to the longitudinal direction, the first absorbent core 111 is located at a position spaced apart from the waist elastic members 23, 33 that are provided in the waist portions 20, 30, and the second absorbent core 112 has the portion that is overlapped with the waist elastic members 23, 33. In the present embodiment, the absorbent core 110 includes the first absorbent core 111 and the second absorbent core 112, thereby increasing the stiffness of the absorbent core 110. However, in the case where the two-layered absorbent core (111, 112) exists in the region where the waist elastic members 23, 33 are provided, there is a risk that the wearer feels discomfort when the diaper 1' is tightly fitted to the waist. Since the waist portions 20, 30 are distanced from the wearer's crotch, high absorption performance is not required as compared with that in the crotch. Thus, the second absorbent core 112 is configured to have the portion that is overlapped with the waist elastic members 23, 33, which reduces discomfort while preventing leakage.

### Others

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof.

### REFERENCE SIGNS LIST

1 diaper (absorbent article)
10 absorbent main body
11 absorbent body
11a absorbent core
11ac constricted portion
11acp narrowest portion
11acpe end
11aCV central region
11ae end
11aSV side region
11b core-wrapping sheet
11ex existence region
11G guide portion
12 top sheet
13 back sheet
14 sheet member
15 second sheet
16 central stretchable member
16be end
16e central stretchable member
16fe end
18 compressed portion
20 front waist portion (waist portion)
21 inner-layer sheet
22 outer-layer sheet
23 waist elastic member
30 back waist portion (waist portion)
30A overlap portion
30B extension portion
31 inner-layer sheet
32 outer-layer sheet
33 waist elastic member
35 extension-portion elastic member
40 waist joining portion
50 leak-proof-wall portion
50A joining portion
50B support point
50E support point
50ie inward end
50ue outward end
51 leak-proof-wall elastic member
110 absorbent core
110CV central region
110ee portion
110SV side region
110vp overlap region
111 first absorbent core
111e outward end
111r core-wrapping sheet
112 second absorbent core
112ac constricted portion
112e outward end
112r core-wrapping sheet
112T low-basis-weight portion

## Claims

1. An absorbent article having a longitudinal direction, a lateral direction, and a thickness direction that intersect each other in an unfolded and stretched state of the absorbent article, the absorbent article comprising:
an absorbent main body including an absorbent core,
in a crotch portion that spans a central position of the absorbent main body in the longitudinal direction, the absorbent core having:
a central region that is located in a central portion of the absorbent core in the lateral direction; and
side regions that are respectively located on two lateral outward sides of the central region,
the absorbent core having a pair of guide portions,
the pair of guide portions being respectively located at boundaries between the central region and the side regions in the lateral direction,
the pair of guide portions extending in the longitudinal direction,
at the central position, a lateral length of the central region being shorter than a lateral length of each of the side regions located on the two lateral outward sides of the central region,
when the absorbent article is put on, each of the guide portions guiding deformation of a region between each of the guide portions and a lateral end of the absorbent core such that the region faces a thigh of a wearer.

2. An absorbent article having a longitudinal direction, a lateral direction, and a thickness direction that intersect each other in an unfolded and stretched state of the absorbent article, the absorbent article comprising:
an absorbent main body including an absorbent core,
in a crotch portion that spans a central position of the absorbent main body in the longitudinal direction, the absorbent core having:
a central region that is located in a central portion of the absorbent core in the lateral direction; and
side regions that are respectively located on two lateral outward sides of the central region,
the absorbent core having a pair of guide portions,
the pair of guide portions being respectively located at boundaries between the central region and the side regions in the lateral direction,
the pair of guide portions extending in the longitudinal direction,
at the central position, a lateral length of the central region being shorter than a lateral length of each of the side regions located on the two lateral outward sides of the central region,
a lateral length of one of the side regions being equal to or less than twice the lateral length of the central region.

3. An absorbent article having a longitudinal direction, a lateral direction, and a thickness direction that intersect each other in an unfolded and stretched state of the absorbent article, the absorbent article comprising:
an absorbent main body including an absorbent core,
in a crotch portion that spans a central position of the absorbent main body in the longitudinal direction, the absorbent core having:
a central region that is located in a central portion of the absorbent core in the lateral direction; and
side regions that are respectively located on two lateral outward sides of the central region,
the absorbent core having a pair of guide portions,
the pair of guide portions being respectively located at boundaries between the central region and the side regions in the lateral direction,
the pair of guide portions extending in the longitudinal direction,
at the central position, a lateral length of the central region being shorter than a lateral length of each of the side regions located on the two lateral outward sides of the central region,
the absorbent core having liquid-absorbent fiber, an existence region referring to a region in the absorbent core where the liquid-absorbent fiber exists,
a non-existence region referring to a region in the absorbent core where the liquid-absorbent fiber does not exist,
the side regions being constituted by only the existence region.

4. The absorbent article according to any one of claims 1 to 3, wherein
at least the side regions have means to increase stiffness of the absorbent core.

5. The absorbent article according to any one of claims 1 to 3, wherein
the side regions have a compressed portion.

6. The absorbent article according to any one of claims 1 to 3, wherein
a central stretchable member that stretches and contracts in the longitudinal direction is provided in a central portion of the crotch portion in the lateral direction, and
the central region has a portion that is overlapped with the central stretchable member as viewed in the thickness direction.

7. The absorbent article according to claim **6,** wherein
the absorbent core has a constricted portion that is located between two longitudinal end portions of the absorbent core,
a lateral length of the constricted portion is shorter than a lateral length of each longitudinal end portion of the absorbent core, and
the central stretchable member is provided in a region where the constricted portion exists with respect to the longitudinal direction.

8. The absorbent article according to claim 6, wherein
a longitudinal one side is a front side of a wearer,
a longitudinal other side is a back side of a wearer, and
in the central stretchable member, a longitudinal length from a front end of the central stretchable member to the central position is longer than a longitudinal length from a back end of the central stretchable member to the central position.

9. The absorbent article according to any one of claims 1 to 3, wherein
the absorbent core includes a first absorbent core and a second absorbent core that is provided on a non-skin side in the thickness direction with respect to the first absorbent core, and
the side regions include an overlap region where the first absorbent core and the second absorbent core are overlapped with each other, as viewed in the thickness direction.

10. The absorbent article according to claim 9, wherein
at least one of the first absorbent core and the second absorbent core has a constricted portion between two longitudinal end portions of the one of the first absorbent core and the second absorbent core,
a lateral length of the constricted portion is shorter than a lateral length of each of the longitudinal end portions of the one of the first absorbent core and the second absorbent core,
the side regions are located in a region where the constricted portion is provided with respect to the longitudinal direction, and
stiffness of the overlap region in the side regions is higher than stiffness of a portion other than the constricted portion.

11. The absorbent article according to claim 9, wherein
in the lateral direction of the side regions, a lateral outward end of the first absorbent core is located outside a lateral outward end of the second absorbent core.

12. The absorbent article according to claim 9, wherein
the second absorbent core has a constricted portion between two longitudinal end portions of the second absorbent core,
a lateral length of the constricted portion is shorter than a lateral length of each of the longitudinal end portions of the second absorbent core, and
the first absorbent core is located in a region where the constricted portion is provided with respect to the longitudinal direction.

13. The absorbent article according to claim 9, wherein
in the central region, a low-basis-weight portion having a lower basis weight than in a surrounding region is provided in at least one of the first absorbent core and the second absorbent core.

14. The absorbent article according to claim 9, wherein
at the central position, a lateral length of the first absorbent core is longer than a lateral length of the second absorbent core, and
at the central position and on a lateral one side, a lateral length from a lateral outward end of the first absorbent core on the lateral one side to a lateral outward end of the second absorbent core on the lateral one side is shorter than a length L1 that is a lateral length of the central region.

15. The absorbent article according to any one of claims 1 to 3, wherein
the absorbent main body has a pair of leak-proof-wall portions on two lateral sides of the absorbent main body,
an elastic member that is contractable in the longitudinal direction is arranged in the pair of leak-proof wall portions, and
at the central position and on a lateral one side, a lateral length from a lateral outward end to a lateral inward end of the leak-proof-wall portion on the lateral one side is equal to or less than half a lateral length from the lateral outward end of the leak-proof-wall portion on the lateral one side to the guide portion on the lateral one side.

16. The absorbent article according to claim 15, wherein
the absorbent core has a constricted portion between two longitudinal end portions of the absorbent core,
a lateral length of the constricted portion is shorter than a lateral length of each of the longitudinal end portions of the absorbent core,
the constricted portion has a narrowest portion where the lateral length of the constricted portion is shortest, and
at the central position and on a lateral one side, the lateral length from the lateral outward end to the lateral inward end of the leak-proof-wall portion on the lateral one side is shorter than a lateral length from the lateral outward end of the leak-proof-wall portion on the lateral one side to an end of the narrowest portion on the lateral one side.

17. The absorbent article according to any one of claims 1 to 3, wherein
the absorbent main body includes:
a top sheet that is located on a skin side in the thickness direction with respect to the absorbent core; and
a second sheet that is provided between the top sheet and the absorbent core,
the central region is located in the region where the second sheet is provided with respect to the lateral direction, and
the central region has a portion that is overlapped with the second sheet as viewed in the thickness direction.

18. The absorbent article according to any one of claims 1 to 3, wherein
the absorbent article includes a waist portion that is arranged around a wearer's waist,
the waist portion includes a waist elastic member that is stretchable and contractable in the lateral direction,
the absorbent core includes a first absorbent core and a second absorbent core that is provided on the non-skin side in the thickness direction with respect to the first absorbent core,
with respect to the longitudinal direction, the first absorbent core is located at a position spaced apart from the waist elastic member, and
with respect to the longitudinal direction, the second absorbent core has a portion that is overlapped with the waist elastic member.
